(19) Europäisches Patentamt
European Patent Office
Office européen des brevets

(11) Veröffentlichungsnummer: **0 007 439**
**B1**

(12) # EUROPÄISCHE PATENTSCHRIFT

(45) Veröffentlichungstag der Patentschrift: **28.07.82**

(51) Int. Cl.³: **C 07 C 143/86** // A01N47/34

(21) Anmeldenummer: **79102054.8**

(22) Anmeldetag: **21.06.79**

(54) **Neue Sulfamidsäurehalogenide und Verfahren zur Herstellung von Sulfamidsäurehalogeniden.**

(30) Priorität: **01.07.78 DE 2828969**
**23.03.79 DE 2911456**

(43) Veröffentlichungstag der Anmeldung:
**06.02.80 Patentblatt 80/3**

(45) Bekanntmachung des Hinweises auf die Patenterteilung:
**28.07.82 Patentblatt 82/30**

(84) Benannte Vertragsstaaten:
**BE CH DE FR GB IT**

(56) Entgegenhaltungen:
**DE-A-1 943 234**
**DE-A-1 953 356**
**DE-A-2 408 530**
**DE-A-2 634 485**
**FR-A-735 765**

**JOURNAL OF CHEMICAL RESEARCH, Miniprint, Heft 10, Oktober 1977, Whitstable, Kent, Great Britain D. BARTHOLOMEW et al. «Synthesis of 2-Alkyl-2-chloro- and fluorosulphonylcarbamoyl chlorides and their reaction with some simple nucleophiles»**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Rote-Turm-Strasse 28, D-6940 Weinheim (DE)**
Erfinder: **Parg, Adolf, Dr., Paray-Le-Monial-Strasse 8, D-6702 Bad Duerkheim 5 (DE)**
Erfinder: **Koenig, Karl-Heinz, Dr., Pierstrasse 8 a, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 007 439 B1

ACTORUM AG

# Neue Sulfamidsäurehalogenide und Verfahren zur Herstellung von Sulfamidsäurehalogeniden

Die Erfindung betrifft neue Sulfamidsäurehalogenide und Verfahren zur Herstellung von Sulfamidsäurehalogeniden durch Umsetzung von monosubstituierten Sulfamidsäurehalogeniden mit Halogenverbindungen in Gegenwart bestimmter Mengen von basischen Verbindungen und in Gegenwart von organischen Lösungsmitteln mit anschliessender Wasserbehandlung bei einem pH von höchstens 7.

Es ist aus der französischen Patentschrift 735 765 bekannt, durch Umsetzung von Acylaniliden mit elementarem Natrium und Folgereaktion mit Sulfurylchlorid N-Acylsulfamidsäurechloride herzustellen. Das Verfahren ist jedoch schwer reproduzierbar, liefert den Endstoff in schlechter Ausbeute und eignet sich wegen der Handhabung von metallischem Natrium nicht für den grosstechnischen Betrieb. Es ist weiterhin bekannt, N-Alkylsulfamidsäurefluoride mittels Acylhalogeniden zu entsprechenden N-Fluorsulfonylverbindungen umzusetzen (DOS 1 943 234). Das Verfahren liefert zwar eine bessere Ausbeute an Endstoff, ist jedoch in der Herstellung der zu verwendenden Alkylsulfamidsäurefluoride und der schwierigen Handhabung des ätzenden und toxischen Fluorwasserstoffs unbefriedigend. So müssen Alkylsulfamidsäurechloride erst mittels Fluorwasserstoff in einer Druckapparatur während 6 Stunden bei 80 bis 90 °C in die entsprechenden Sulfamidsäurefluoride umgewandelt werden, wobei Destillationsprozesse zur Abtrennung der überschüssigen Fluorwasserstoffsäure und Reinigung der Sulfamidsäurefluoride erforderlich sind. Die im Falle des Methylsulfamidsäurefluorids erzielte Ausbeute liegt beispielsweise bei 70 Prozent. Es ist auch eine Umsetzung des N-Äthylsulfamidsäurechlorids selbst mit Difluorchlormethansulfenylchlorid zu der entsprechenden Sulfamidsäurechloridverbindung beschrieben (DOS 1 953 356), die jedoch nur in 48 Prozent Ausbeute erhalten wird. In J. chem. Res. (M) 1977, Seiten 2801 bis 2809 wird die Umsetzung von Sulfamidsäurehalogeniden mit Phosgen in Gegenwart von Pyridin und mit Toluol als Lösungsmittel zu N-Alkyl-N-chlorsulfonylcarbamoylchloriden beschrieben, wobei der Aufarbeitung des Reaktionsgemisches ein Filtrationsprozess über ein Hyflo super cel-Filter vorgeschaltet werden muss. Anschliessend wird das Filtrat fraktioniert destilliert. Führt man die Aufarbeitung ohne den Filtrationsprozess durch, erhält man keine wesentlichen Mengen an reinem Endstoff. Die Umsetzung und ihre Aufarbeitung werden stets unter Verwendung organischer Lösungsmittel und in Abwesenheit von Wasser duchgeführt. Aus den Carbamidsäurechloriden können in einer weiteren Stufe durch Umsetzung mit Alkoholen die entsprechenden Carbamidsäureester hergestellt werden. Bei grösseren Umsätzen verstopfen sich die Filter häufig, und grosse Mengen Hyflo super cel müssen verwendet werden. Für einen wirtschaftlichen und grosstechnischen Prozess kommt das Verfahren aus vorgenannten Gründen und wegen der erforderlichen Verbrennung und Deponie des Filterrückstandes nicht in Betracht.

In der deutschen Offenlegungsschrift 2 408 530 wird ein Verfahren zur Herstellung von N-β-Halogenalkyl-N-alkylaminosulfonylhalogeniden durch Umsetzung von Aziridinen mit Sulfurylhalogeniden beschrieben. Wie die Beispiele zeigen, setzt man in Abwesenheit einer zusätzlichen Base und von Wasser unter Verwendung von organischen Lösungsmitteln um. Das Verfahren erlaubt die Synthese durch Halogenatome, Acylgruppen oder Heteroatome substituierter Sulfamidsäurechloride nicht.

In der deutschen Offenlegungsschrift 2 634 485 wird ein Verfahren zur Herstellung von Sulfamidsäurehalogeniden durch Umsetzung von N-(α-Halogenalkyl)-suflamidsäurehalogeniden mit Alkoholen beschrieben. Zweckmässig verwendet man Basen als Katalysatoren. Die Umsetzung und Aufarbeitung wird in Abwesenheit von Wasser unter Verwendung organischer Lösungsmittel durchgeführt. Das Verfahren erlaubt nur die Synthese von Alkoxymethyl-sulfamidsäurehalogeniden, jedoch ohne N-Acylgruppen bzw. Heteroatome als Substituenten. Das Verfahren ist aufwendig und zweistufig.

Alle vorgenannten Verfahren sind mit Bezug auf Ausbeute, leicht zugängliche Ausgangsstoffe, Reinheit und Umfang der herzustellenden Endstoffe, einfachen, wirtschaftlichen Betrieb und Umweltfreundlichkeit, gerade auch im grosstechnischen Massstab, unbefriedigend.

Es wurde nun gefunden, dass man Sulfamidsäurehalogenide der Formel

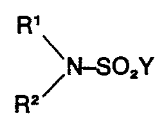 I,

in der $R^1$ einen aliphatischen oder cycloaliphatischen Rest bedeutet, $R^2$ den Rest

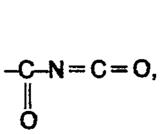, 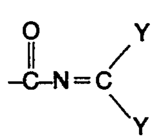, 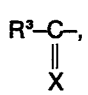, 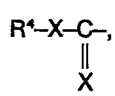, 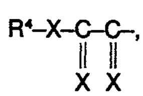, 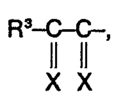, 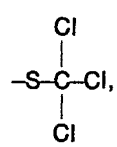, 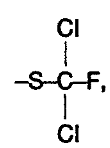,

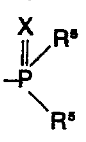 $-CH_2XR^6$, 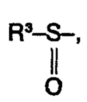, $R^3-S-S-$ oder 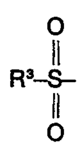

bezeichnet, $R^3$ und $R^4$ jeweils für einen aliphatischen, cycloaliphatischen, araliphatischen, aromatischen oder heterocyclischen Rest stehen, $R^3$ auch ein Halogenatom bezeichnen kann, die einzelnen Reste $R^5$ gleich oder verschieden sein können und jeweils ein Halogenatom, den Rest $-X-R^6$ oder $-R^6$ bedeuten, $R^6$ für einen aliphatischen Rest steht, die einzelnen Reste X gleich oder verschieden sein können und jeweils ein Sauerstoffatom oder ein Schwefelatom bedeuten, die einzelnen Rest Y gleich oder verschieden sein können und jeweils für ein Halogenatom stehen, durch Umsetzung von Sulfamidsäurehalogeniden und Halogenverbindungen in Gegenwart von Basen und Lösungsmitteln, vorteilhaft erhält, wenn man monosubstituierte Sulfamidsäurehalogenide der Formel

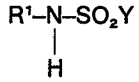 II,

in der $R^1$ und Y die vorgenannten Bedeutungen besitzen, mit Halogenverbindungen der Formel

$$R^2-Y \quad III,$$

in der $R^2$ und Y die vorgenannten Bedeutungen besitzen, in Gegenwart einer basischen Verbindung in einer Menge von 1 bis 1,5 Äquivalenten je Mol Ausgangsstoff II und von inerten, organischen Lösungsmitteln umsetzt und anschliessend das so gebildete Reaktionsgemisch mit Wasser bei einem pH von höchstens 7 behandelt.

Weiterhin wurden die neuen Sulfonamidsäurehalogenide der Formel

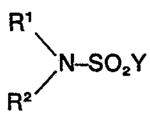 I,

in der $R^1$ einen Alkylrest oder einen durch mehrere Fluor- und/oder Chloratome, oder durch ein Fluoratom oder Chloratom in β-, γ- und/oder δ-Stellung zum Stickstoffatom substituierten Alkylrest mit jeweils 1 bis 8 Kohlenstoffatomen oder einen Cycloalkylrest mit 4 bis 8 Kohlenstoffatomen bedeutet, $R^2$ den Rest

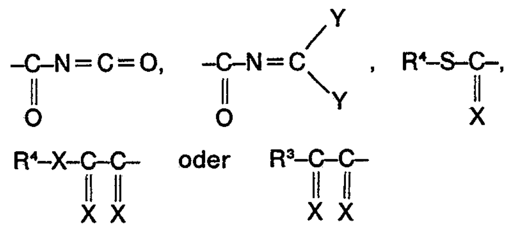

bezeichnet, darüber hinaus, wenn Y Chlor bedeutet, $R^2$ auch für 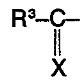 stehen kann, $R^3$ und $R^4$ jeweils für einen unsubstituierten oder einen durch ein oder zwei Fluor--oder Chloratome substituierten Alkylrest mit jeweils 1 bis 8 Kohlenstoffatomen, oder einen gegebenenfalls durch ein oder 2 Halogenatome, Nitro- und/oder Alkoxygruppen mit 1 bis 3 Kohlenstoffatomen substituierten Arylrest mit 6 bis 12 Kohlenstoffatomen stehen, $R^3$ auch, wenn Y Chlor und $R^1$ Äthyl, n-Propyl, n-Butyl, sek.-Butyl bedeuten, ein Chloratom bezeichnen kann, die einzelnen Reste X gleich oder verschieden sein können und jeweils ein Sauerstoffatom oder ein Schwefelatom bedeuten, die einzelnen Reste Y gleich oder verschieden sein können und jeweils für ein Fluor- oder Chloratom stehen.

Die Umsetzung lässt sich für den Fall der Verwendung von Isopropylsulfamidsäurechlorid und Chlorameisensäuremethylester durch folgende Formeln wiedergeben:

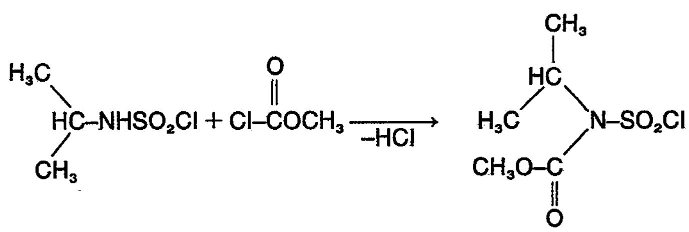


Die Umsetzung lässt sich für den Fall der Verwendung von Isopropylsulfamidsäurechlorid und Chlorcarbonylisocyanat durch folgende Formeln wiedergeben:

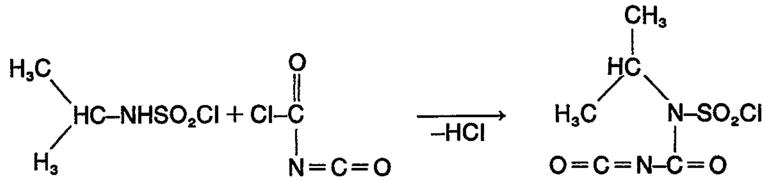


Im Hinblick auf den Stand der Technik liefert das erfindungsgemässe Verfahren auf einfachere und wirtschaftlichere Weise eine grosse Zahl neuer und bekannter N,N-disubstituierter Sulfamidsäurehalogenide in besserer Ausbeute und Reinheit. Die Reaktionszeit ist kurz, die Aufarbeitung des Reaktionsgemisches, gerade auch im Hinblick auf den Umweltschutz, einfach und betriebssicher. Auch Ausgangsstoffe II mit Alkylgruppen höherer Zahl an Kohlenstoffatomen und gegebenenfalls mit Halogenatomen als Substituenten können nach dem erfindungsgemässen Verfahren umgesetzt werden. Alle diese vorteilhaften Ergebnisse sind im Hinblick auf den Stand der Technik überra-

schend. Nicht vorhersehbar war es im Hinblick auf die deutsche Offenlegungsschrift 1 943 234, dass sich vorteilhaft die Sulfamidsäurechloride ohne vorherige Umwandlung in ihre Sulfamidsäurefluoride umsetzen lassen. Wegen des hochgiftigen Charakters disubstituierter Sulfamidsäurefluoride (vergleiche Houben-Weyl, Methoden der Organischen Chemie, Band 11/2, Seite 703) zeigt das erfindungsgemässe Verfahren so besondere Umweltfreundlichkeit, im Vergleich zu dem Verfahren der deutschen Offenlegungsschrift 1 953 356 bessere Ausbeuten. Überraschend ist es auch ohne Verwendung von Hyflo super cel-Filter durchführbar. Im Falle von Chlorcarbonylsulfamidsäurechloriden liefert das erfindungsgemässe Verfahren die gewünschten Endstoffe in grösserer Ausbeute und Reinheit und ohne Belastung der Umwelt.

Ein saurer oder neutraler, wässriger Waschprozess ist insbesondere, wenn $R^2$ den Rest

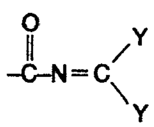

bezeichnet, für das Gelingen der Reaktion von Bedeutung.

Überraschend ist ebenfalls die Durchführbarkeit eines sauren oder neutralen, wässrigen Waschprozesses, der für das Gelingen der Reaktion in allen Fällen von Ausgangsstoffen, die die weiteren Bedeutungen von $R^2$ besitzen, von entscheidender Bedeutung ist. Unterbleibt beispielsweise im Falle der Umsetzung eines Alkylsulfamidsäurechlorids mit Chlorameisensäuremethylester die Nachbehandlung mit Wasser, so erhält man bei der anschliessenden Destillation auch unter schonenden Bedingungen nur eine geringe Menge niedrigsiedender Zersetzungsprodukte neben einem zum überwiegenden Teil nicht destillierbaren zähen Reaktionsrückstand. Die Durchführbarkeit dieses erfindungsgemässen Waschprozesses war nicht vorherzusehen, denn Sulfamidsäurefluoride sind zwar gegenüber Wasser in begrenztem Masse stabil, Sulfamidsäurechloride zersetzen sich jedoch in Gegenwart von Wasser ausserordentlich heftig. Eine hohe Hydrolyseempfindlichkeit beschreiben schon Acta Chem. Scand., Band 17 (1963), Seiten 2142 («they react very rapidly with water») und Ann., Band 729, Seite 44 (1969) («mit Wasserreagieren sie stürmisch und exotherm»).

Die Ausgangsstoffe III können nach bekannten Verfahren, z.B. durch Umsetzung von Halogencyanen mit Kohlensäuredihalogeniden nach der in der Angewandten Chemie, Band 89, Seiten 789 bis 796 (1977) beschriebenen Arbeitsweise, hergestellt werden.

Die Ausgangsstoffe II können mit den Ausgangsstoffen III in stöchiometrischer Menge oder mit einem Überschuss an Ausgansstoff III umgesetzt werden, vorzugsweise in einem Verhältnis von 1 bis 1,5 Mol, insbesondere 1 bis 1,1 Mol Ausgangsstoff III je Mol Ausgangsstoff II.

Bevorzugte Ausgangsstoffe II und III und dementsprechend bevorzugte Endstoffe I sind solche, in deren Formeln $R^1$ einen geradkettigen oder verzweigten Alkylrest oder einen durch mehrere Halogenatome, insbesondere Fluor- und/oder Chloratome, oder durch ein Halogenatom, insbesondere Fluoratom oder Chloratom, zweckmässig in β-, γ- und/oder δ-Stellung zum Stickstoffatom substituierten Alkylrest mit jeweils 1 bis 20, insbesondere jeweils 1 bis 8 Kohlenstoffatomen oder einen Cycloalkylrest mit 4 bis 8 Kohlenstoffatomen bedeutet, $R^2$ den Rest

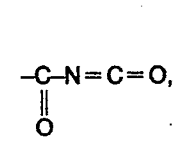, 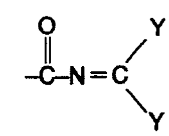, 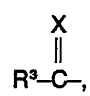, 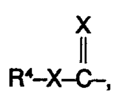, 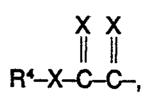, 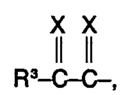, 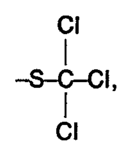, 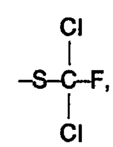,

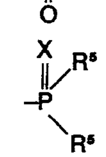 $-CH_2XR^6$, 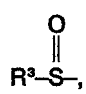, $R^3-S-S-$ oder 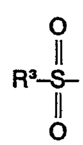

bezeichnet, $R^3$ und $R^4$ jeweils für einen unsubstituierten oder einen durch eine oder zwei Äthergruppen, zweckmässig mit 1 bis 4 Kohlenstoffatomen in der Alkoxygruppe, und/oder ein oder zwei Halogenatome, vorzugsweise Fluor- oder Chloratome substituierten Alkylrest mit jeweils 1 bis 20, bevorzugt jeweils 1 bis 8 Kohlenstoffatomen (die Kohlenstoffatome der Äthergruppen nicht eingerechnet), oder einen unsubstituierten oder durch eine oder zwei Äthergruppen, zweckmässig mit 1 bis 4 Kohlenstoffatomen in der Alkoxygruppe, und/oder ein oder zwei Halogenatome, vorzugsweise Fluoratome oder Chloratome, substituierten, geradkettigen oder verzweigten Alkenylrest oder Alkinylrest mit jeweils 2 bis 20, insbesondere 2 bis 8 Kohlenstoffatomen (die Kohlenstoffatome der Äthergruppen nicht eingerechnet), oder einen unsubstituierten oder durch ein Chloratom substituierten Cycloalkylrest mit 4 bis 8 Kohlenstoffatomen oder einen gegebenenfalls durch 1 oder 2 Halogenatome, Nitro- und/oder Alkoxygruppen mit 2 bis 3 Kohlenstoffatomen substituierten Aralkylrest oder Alkylarylrest mit 7 bis 12 Kohlenstoffatomen (die Kohlenstoffatome der Äthergruppen nicht eingerechnet), oder einen gegebenenfalls durch ein oder 2 Halogenatome, Nitro- und/oder Alkoxygruppen mit 1 bis 3 Kohlenstoffatomen substituierten Arylrest mit 6 bis 12 Kohlenstoffatomen (die Kohlenstoffatome der Äthergruppen nicht eingerechnet), oder einen gegebenenfalls durch ein oder 2 Halogenatome, Nitro- und/oder Alkoxygruppen mit 1 bis 3 Kohlenstoffatomen substituierten 5- oder 6gliedrigen heterocyclischen Rest, der ein oder zwei Stickstoffatome und/oder ein Sauerstoffatom enthalten kann, stehen $R^3$ auch ein Fluoratom oder insbesondere ein Chloratom bezeichnen kann, die einzelnen Reste $R^5$ gleich oder verschieden sein können und jeweils ein Chloratom, den Rest $-X-R^6$ oder $-R^6$ bedeuten, $R^6$ für einen Alkylrest mit 1 bis 20, insbesondere 1 bis 8, vorzugsweise 1 bis 3 Kohlenstoffatomen steht, die einzelnen Rest X gleich oder verschieden sein können und jeweils ein Sauerstoffatom oder ein Schwefelatom bedeuten und die einzelnen Reste Y gleich oder verschieden sein können und jeweils für ein Bromatom, ein Fluoratom oder insbesondere ein Chloratom stehen.

Die genannten Reste können noch durch unter den Reaktionsbedingungen inerte Gruppen, z.B. Alkylgruppen mit 1 bis 4 Kohlenstoffatomen, Carbalkoxygruppen mit 2 bis 4 Kohlenstoffatomen oder eine Chlorcarbonylgruppe substituiert sein.

Bevorzugt kommen als Ausgangsstoffe II in Betracht: Methylsulfamidsäurechlorid,
Äthylsulfamidsäurechlorid,
n-Propylsulfamidsäurechlorid,
Isopropylsulfamidsäurechlorid,
n-Butylsulfamidsäurechlorid,
sek.-Butylsulfamidsäurechlorid,
Isobutylsulfamidsäurechlorid,
tert.-Butylsulfamidsäurechlorid
Pentylsulfamidsäurechlorid,
Cyclopentylsulfamidsäurechlorid,
n-Hexylsulfamidsäurechlorid,
Cyclohexylsulfamidsäurechlorid,
n-Heptylsulfamidsäurechlorid,
1,2-Dimethylbutyl-(1)-sulfamidsäurechlorid,
1,3-Dimethylbutyl-(1)-sulfamidsäurechlorid,
3-Chlorpentyl-(1)-sulfamidsäurechlorid,
3-Chlorpropyl-(1)-sulfamidsäurechlorid,
4-Chlorisoamyl-(1)-sulfamidsäurechlorid,
2-Chlormethylpropyl-(1)-sulfamidsäurechlorid,
2-Fluormethylpropyl-(1)-sulfamidsäurechlorid,
1-Chlorpropyl-(2)-sulfamidsäurechlorid,
2-Chlorpropyl-(1)-sulfamidsäurechlorid,
2-Chlorisobutyl-(1)-sulfamidsäurechlorid,
1-Chlorbutyl-(2)-sulfamidsäurechlorid,
3-Chlorbutyl-(2)-sulfamidsäurechlorid,
2-Fluoräthyl-(1)-sulfamidsäurechlorid,
1-Fluorpropyl-(2)-sulfamidsäurechlorid,
2-Fluorpropyl-(1)-sulfamidsäurechlorid,
1-Fluorbutyl-(2)-sulfamidsäurechlorid,
2-Fluorbutyl-(1)-sulfamidsäurechlorid,
2-Fluorisobutyl-(1)-sulfamidsäurechlorid,
3-Fluorbutyl-(1)-sulfamidsäurechlorid
4-Chlorpentyl-(1)-sulfamidsäurechlorid,
4-Chlorbutyl-(2)-sulfamidsäurechlorid,
Chlor-tert.-butyl-sulfamidsäurechlorid;
entsprechende Sulfamidsäurefluoride,
z.B. Methylsulfamidsäurefluorid,
Äthylsulfamidsäurefluorid,
n-Propylsulfamidsäurefluorid,
Isopropylsulfamidsäurefluorid,
n-Butylsulfamidsäurefluorid,
Isobutylsulfamidsäurefluorid,
tert.-Butylsulfamidsäurefluorid,
sek.-Butylsulfamidsäurefluorid,
Pentylsulfamidsäurefluorid,
Cyclopentylsulfamidsäurefluorid,
n-Hexylsulfamidsäurefluorid,
Cyclohexylsulfamidsäurefluorid,
n-Heptylsulfamidsäurefluorid,
1,2-Dimethylbutyl-(1)-sulfamidsäurefluorid,
1,3-Dimethylbutyl-(1)-sulfamidsäurefluorid.

Vorteilhaft werden als Ausgangsstoffe III verwendet: Acetylchlorid,
Propionylchlorid,
Butyrylchlorid,
Valerylchlorid,
Isovalerylchlorid,
sek.-Valerylchlorid,
Capronsäurechlorid,
α-Methylvaleriansäurechlorid,
Caprylsäurechlorid,
Önanthsäurechlorid,
Pelargonsäurechlorid,
Caprinsäurechlorid,
Undecanoylchlorid,
Dodecanoylchlorid,
Tridecanoylchlorid,
Tetradecanoylchlorid,
Pentadecanoylchlorid,
Hexadecanoylchlorid,
Acetoxyacetylchlorid,
Chloracetylchlorid,
Dichloracetylchlorid,
α-Chlorpropionylchlorid,
α,α-Dichlorpropionylchlorid
α-Chlorbutyrylchlorid;
analoge Carbonsäurefluoride;
α-Chlorvaleriansäurechlorid,
α-Chlorcapronsäurechlorid,
β-Chlorpropionsäurechlorid,
Pyridincarbonsäurechlorid-(2),
Pyrrolcarbonsäurechlorid-(2),
Piperidin-carbonsäurechlorid-(2),
Piperazin-carbonsäurechlorid-(2),
Furancarbonsäurechlorid-(2),
Fluoracetylchlorid,
Acryloylchlorid,
But-(2)-enoyl-(1)-chlorid,
Pent-(3)-enoyl-(1)-chlorid,
β-Methoxypropionylchlorid,
γ-Methoxybutyrylchlorid,
Cyclopentanoylchlorid,
Cyclohexanoylchlorid,
Benzoylchlorid,
α-Naphthoylchlorid,
o-, m-, p-Chlorbenzoylchlorid,
o-, m-, p-Nitrobenzoylchlorid,
Phosgen,
Chlorcarbonylfluorid,
Difluorphosgen,
Thiophosgen,
Oxalylchlorid,
Phenylacetylchlorid,
analoge Thiocarbonsäurechloride;
Methoxymethylchlorid,
Äthoxymethylchlorid,
Methylthiomethylchlorid;
Chlorameisensäuremethylester,

Chlorameisensäureäthylester,
Chlorameisensäureisopropylester,
Chlorameisensäure-n-propylester,
Chlorameisensäurebutylester,
Chlorameisensäureisobutylester,
Chlorameisensäure-sek.-butylester,
Chlorameisensäurephenylester;
analoge Oxalsäuremonochloridmonoalkylester;
analoge Thioester;
analoge Dithioester;
Dischwefeldichlorid,
Thionylchlorid,
Thionylfluorid,
Sulfurylchlorid,
Sulfurylfluorid,
Methylsulfonylchlorid,
Äthylsulfonylchlorid,
n-Propylsulfonylchlorid,
Isopropylsulfonylchlorid,
n-Butylsulfonylchlorid,
sek.-Butylsulfonylchlorid,
Benzolsulfonylchlorid,
o-, m-, p-Toluolsulfonylchlorid,
o-, m-, p-Chlorbenzolsulfonylchlorid,
o-, m-, p-Nitrobenzolsulfonylchlorid,
β-Naphthalinsulfonylchlorid;
analoge Sulfonylfluoride;
Trichlormethylschwefelchlorid,
Monofluordichlormethylschwefelchlorid;
O,O-Dimethylphosphorsäurechlorid,
O,O-Diäthylphosphorsäurechlorid,
O-Methyl-O-äthylphosphorsäurechlorid,
O,S-Dimethylmonothiophosphorylchlorid,
O-Äthyl-S-n-propylmonothiophosphorylchlorid,
Phosphoroxychlorid,
Thiophosphorylchlorid,
Dimethylphosphinsäurechlorid,
Diäthylphosphinsäurechlorid,
Äthylphosphinsäuredichlorid,
O-Methylphosphorsäuredichlorid,
Chlorcarbonylisocyanat,
N-Chlorcarbonyl-isocyaniddichlorid,
N-Fluorcarbonyl-isocyaniddichlorid.

Die Reaktion wird in der Regel bei einer Temperatur von −50 bis +80°C, im Falle von Ausgangsstoffen III, worin $R^2$ den Rest

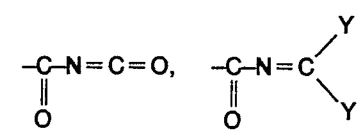

bedeutet, zweckmässig zwischen −50 und +70°C, vorteilhaft von −30 bis +60°C, vorzugsweise von −10 bis +40°C, insbesondere von 0 bis 30°C, im Falle aller übrigen Ausgangsstoffe III vorzugsweise von −10 bis +40°C, insbesondere von 0 bis 30°C, drucklos oder unter Druck, kontinuierlich oder diskontinuierlich durchgeführt. Man verwendet unter den Reaktionsbedingungen inerte, organische Lösungsmittel. Als Lösungsmittel kommen z.B. in Frage:
Halogenkohlenwasserstoff,
insbesondere Chlorkohlenwasserstoffe,
z.B. Tetrachloräthylen,
1,1,2,2- oder 1,1,1,2-Tetrachloräthan,
Amylchlorid, Cyclohexylchlorid,
Dichlorpropan, Methylenchlorid,
Dichlorbutan, Isopropylbromid,
n-Propylbromid, Butylbromid,
Chloroform, Äthyljodid,
Propyljodid, Chlornaphthalin,
Dichlornaphthalin, Tetrachlorkohlenstoff,
1,1,1- oder 1,1,2-Trichloräthan,
Trichloräthylen, Pentachloräthan,
o-, m-, p-Difluorbenzol,
1,2-Dichloräthan,
1,1-Dichloräthan, n-Propylchlorid,
1,2-cis-Dichloräthylen,
n-Butylchlorid,
2-, 3- und iso-Butylchlorid,
Chlorbenzol, Fluorbenzol,
Brombenzol, Jodbenzol,
o-, p- und m-Dichlorbenzol,
o-, p-, m-Dibrombenzol,
o-, m-, p-Chlortoluol.
1,2,4-Trichlorbenzol, 1,10-Dibromdekan,
1,4-Dibrombutan; Äther,
z.B. Äthylpropyläther,
Methyl-tert.-butyläther,
n-Butyläthyläther, Di-n-butyläther,
Diisobutyläther, Diisoamyläther,
Diisopropyläther, Anisol,
Phenetol, Cyclohexylmethyläther,
Diäthyläther,
Äthylenglykoldimethyläther,
Tetrahydrofuran, Dioxan,
Thioanisol,
β,β'-Dichlordiäthyläther;
Nitrokohlenwasserstoffe wie Nitromethan,
Nitroäthan, Nitrobenzol,
o-, m-, p-Chlornitrobenzol,
o-Nitrotoluol;
Nitrile wie Acetonitril,
Butyronitril, Isobutyronitril,
Benzonitril, m-Chlorbenzonitril;
aliphatische oder cycloaliphatische
Kohlenwasserstoffe,
z.B. Heptan, Pinan, Nonan,
o-, m-, p-Cymol,
Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; aromatische Kohlenwasserstoffe, z.B. Benzol, Toluol, o-, m-, p-Xylol, Tetralin, 1,3,5-Trimethylbenzol; und entsprechende Gemische. Zweckmässig verwendet man das Lösungsmittel in einer Menge von 100 bis 2000 Gewichtsprozent, vorzugsweise von 400 bis 800 Gewichtsprozent, bezogen auf Ausgangsstoff II.

Die Umsetzung wird in Gegenwart einer basischen Verbindung in einer Menge von 1 bis 1,5, vorzugsweise von 1 bis 1,2 Äquivalenten basischer Verbindung, bezogen auf ein Mol Ausgangsstoff II, durchgeführt. Bevorzugte basische Verbindungen sind Alkaliverbindungen, Erdalkaliverbindungen, Ammoniumverbindungen und insbesondere tertiäre Amine sowie entsprechende Gemische. Es können aber auch Zinkverbindungen verwendet werden. Es kommen z.B. als basische Verbindungen in Frage:
Kaliumhydroxid, Natriumhydroxid,
Kaliumcarbonat, Natriumcarbonat,
Lithiumhydroxid, Lithiumcarbonat,
Natriumbicarbonat, Kaliumbicarbonat,
Calciumhydroxid, Calciumoxid,
Bariumoxid, Magnesiumhydroxid,
Magnesiumoxid, Bariumhydroxid,
Calciumcarbonat, Magnesiumcarbonat,
Magnesiumbicarbonat, Magnesiumacetat,
Zinkhydroxid, Zinkoxid,
Zinkcarbonat, Zinkbicarbonat,
Zinkacetat, Natriumformiat,
Natriumacetat, Natriumpropionat,
Natriumbutyrat, Natriumisobutyrat,
Kaliumformiat, Kaliumacetat,
Kaliumpropionat, Kaliumbutyrat,
Kaliumisobutyrat,
Kalium-tert.-butylat,
Trimethylamin, Triäthylamin,
Tripopylamin, Triisopropylamin,
Tributylamin, Triisobutylamin,
Tri-sek.-butylamin,
Tri-tert.-butylamin, Tribenzylamin,
Tricyclohexylamin, Triamylamin,
Trihexylamin, N,N-Dimethylanilin,
N,N-Diäthylanilin, N,N-Dipropylanilin,
N,N-Dimethyltoluidin, N,N-Diäthyltoluidin,
N,N-Dipropyltoluidin,
N,N-Dimethyl-p-aminopyridin,
N,N,Diäthyl-p-aminopyridin,
N,N-Dipropyl-p-aminopyridin,
N-Methylpyrrolidon, N-Äthylpyrrolidon,
N-Methylpiperidin, N-Äthylpiperidin,
N-Methylpyrrolidin, N-Äthylpyrrolidin,
N-Methylimidazol, N-Äthylimidazol,
N-Methylpyrrol, N-Äthylpyrrol,
N-Methylmorpholin, N-Äthylmorpholin,
N-Methylhexamethylenimin,
N-Äthylhexamethylenimin, Pyridin,
Chinolin, α-Picolin, β-Picolin,
γ-Picolin, Isochinolin, Pyrimidin,
Acridin, N,N,N',N'-Tetramethyläthylendiamin,
N,N,N',N'-Tetraäthyläthylendiamin,
Chinoxalin, Chinazolin,
N-Propyldiisopropylamin,
N,N-Dimethylcyclohexylamin,
2,6-Lutidin, 2,4-Lutidin,
Trifurfurylamin, Triäthylendiamin.

Die Reaktion kann wie folgt durchgeführt werden: Ein Gemisch von Ausgangsstoff II und III, basischer Verbindung und organischem Lösungsmittel wird während 0,1 bis 6 Stunden, im Falle von Ausgangsstoffen III, worin $R^2$ den Rest

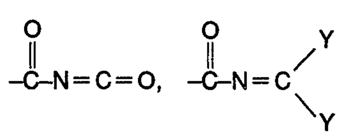

bedeutet, zweckmässig während 0,3 bis 6 Stunden, vorzugsweise 0,5 bis 3 Stunden, in allen übrigen Fällen während 0,1 bis 5 Stunden, vorzugsweise 0,2 bis 1 Stunden, bei der Reaktionstemperatur gehalten. Man kann z.B. den Ausgangsstoff II mit dem Ausgangsstoff III zusammen mit Lösungsmittel vorlegen und dann die basische Verbindung zugeben und bei der Reaktionstemperatur halten. In einer bevorzugten Form des erfindungsgemässen Verfahrens vermischt man den Ausgangsstoff III mit der basischen Verbindung während 10 bis 30 Minuten in einem inerten, organischen Lösungsmittel bei −50 bis +40 °C, vorzugsweise −10 bis +30 °C, und gibt dann den Ausgangsstoff II bei gleicher Temperatur innerhalb 10 bis 30 Minuten hinzu. Zur Beendigung der Umsetzung rührt man noch 0,1 bis 4 Stunden bei 0 bis 40 °C, insbesondere 0,2 bis 0,3 Stunden bei 10 bis 30 °C, nach. Im Falle von Ausgangsstoffen III, worin $R^2$ den Rest

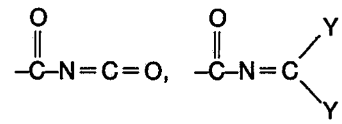

bedeutet, in einer bevorzugten Form des erfindungsgemässen Verfahrens vermischt man den Ausgangsstoff III mit der basischen Verbindung während 10 bis 30 Minuten in einem inerten, organischen Lösungsmittel bei −50 bis +40 °C, vorzugsweise −20 bis +30 °C, und gibt dann den Ausgangsstoffen II bei gleicher Temperatur innerhalb 10 bis 30 Minuten hinzu. Zur Beendigung der Umsetzung rührt man noch 0,1 bis 5 Stunden bei 0 bis 45 °C, insbesondere 0,2 bis 2 Stunden bei 10 bis 30 °C nach.

Das so erhaltene Reaktionsgemisch kann gegebenenfalls noch filtriert und der Filterrückstand mit einem der vorgenannten Lösungsmittel, z.B. Methylenchlorid oder Toluol, ausgewaschen werden. Anschliessend wird der Endstoff im Falle von Ausgangsstoffen III, worin $R^2$ den Rest

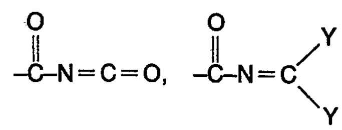

bedeutet, in üblicher Weise, z.B. durch Abtrennung der organischen Phase und fraktionierte Destillation dieser Phase, isoliert. Man führt bevorzugt die Wasserbehandlung sofort nach Beendigung der Reaktion durch. Man handelt zweckmässig das Reaktionsgemisch bzw. vorgenanntes Filtrat während 2 bis 30 Minuten bei einer Temperatur von 0 bis 30 °C, insbesondere 5 bis 15 °C, bei einem pH von 1 bis 7, vorzugsweise von 1 bis 5, insbesondere von 1 bis 3, drucklos oder unter Druck, kontinuierlich oder diskontinuierlich. Die Wasserbehandlung wirkt wie eine Extraktion, und es bilden sich 2 Phasen. Anschliessend wird der Endstoff in üblicher Weise, z.B. durch Abtrennung der organischen Phase und fraktionierte Destillation dieser Phase, isoliert.

Nach einer Behandlung mit Wasser ist der so erhaltene rohe Endstoff aber in vielen Fällen bereits so rein, dass er nach Abzug des Lösungsmittels direkt für weitere Umsetzungen eingesetzt werden kann.

Die nach dem Verfahren der Erfindung herstellbaren, teilweise neuen Verbindungen sind wertvolle Ausgangsstoffe für die Herstellung von Pflanzenschutzmitteln, Farbstoffen und Pharmazeutika. Beispielsweise gelangt man durch Umsetzung des N-Acetyl-N-methyl-aminosulfamidsäurechlorids mit 2,3-Dihydro-3,3-dimethyl-2-äthoxy-5-hydroxy-benzofuran in Äther in Gegenwart äquivalenter Mengen Triäthylamin in glatter Reaktion direkt zu dem 2,3-Dihydro-3,3-dimethyl-2-äthoxybenzofuran-5-yl-N-methyl-carbonyl-N-methyl-aminosulfonat ($n_D^{25}$ = 1,5042), das bisher nur zunächst durch Herstellung des Methylaminosulfamidsäureesters und nachträglicher Acetylierung hergestellt wird (DOS 2 402 370); diese Stoffe besitzen ausgezeichnete herbizide Wirkung. In analoger Weise gelangt man durch Umsetzung mit N-Alkylsulfonyl-N-alkyl-sulfamidsäurechloriden oder N-Alkoxycarbonyl-N-alkylsulfamidsäurechloriden zu entsprechend am Stickstoffatom substituierten 2,3-Dihydro-3,3-dimethyl-3-alkoxybenzofuran-5-yl-amino-sulfonaten, deren ausgezeichnete herbizide Wirkung DOS 2 402 370 beschreibt. Weitere Verwendungsmöglichkeiten sind in dem Beispiel 32 aufgeführt.

Beispielsweise gelangt man durch Umsetzung der N-Carbonylisocyanato-N-alkyl-(cycloalkyl)-sulfamidsäurechloride mit Alkylaminen, z.B. Isopropylamin, zu den N-Alkyl-N'-(N''-methyl-N''-chlorsulfonyl)-carbamidoharnstoffen; diese Stoffe besitzen ausgezeichnete herbizide Wirkung. In analoger Weise gelangt man durch Umsetzung von N-Carbonylisocyanato-N-alkyl-(N-cycloalkyl)-sulfamidsäurechloriden mit substituierten Anilinen zu entsprechend an den beiden endständigen Stickstoffatomen substituierten Biureten, die durch stark alkalische Hydrolyse zu den in DE-AS 1 032 595 beschriebenen, herbiziden Biureten umgewandelt werden. Entsprechend kann man die N-Carbonylisocyaniddichlorido-N-alkyl-(N-cycloalkyl)-sulfamidsäurechloride nach Umwandlung mittels saurer, wässriger Hydrolyse in die vorgenannten N-Carbonylisocyanato-Verbindungen I in die vorgenannten Biurete umwandeln. Weitere Verwendungsmöglichkeiten sind in dem Beispiel 44 aufgeführt.

Die in den folgenden Beispielen aufgeführten Teile bedeuten Gewichtsteile.

Beispiel 1

a) 21,3 Teile Triäthylamin in 40 Teilen Toluol werden bei 10 °C in eine Lösung von 36,5 Teilen Dichlorfluormethylsulfenylchlorid in 90 Teilen Toluol eingegeben. Anschliessend werden 30,2 Teile Äthylsulfamidsäurechlorid bei 18 °C innerhalb 10 Minuten zugegeben und das Reaktionsgemisch eine halbe Stunde bei 22 °C gerührt. Nun wird das Reaktionsgemisch während 5 Minuten mit 300 Teilen Wasser bei 10 °C und einem pH von 1 gerührt. Dann wird aus dem gebildeten 2-phasigen Gemisch die organische Phase abgetrennt, filtriert und fraktioniert destilliert. Man erhält 38,1 Teile (68,9% der Theorie) N-Dichlorfluormethylthio-N-äthylsulfamidsäurechlorid vom Kp 65 bis 76 °C/0,08 mbar und $n_D^{25}$ = 1,4976.

b) (Vergleich):

Die Umsetzung wird analog Beispiel 1a) ohne Wasserbehandlung durchgeführt. Nach Beendigung der Reaktion wird das Gemisch abgesaugt, der Filterrückstand mit Toluol nachgewaschen, das Filtrat eingeengt und destilliert. Man erhält 11,9 Teile (21,6% der Theorie) Endstoff I vom Kp 81 bis 105 °C/0,13 mbar und $n_D^{25}$ = 1,5020.

c) (Vergleich):

Die Umsetzung wird analog Beispiel 1b) durchgeführt, jedoch erfolgt die Zugabe der Base zu der Mischung der beiden Ausgangsstoffe. Man erhält 5,17 Teile (9,4% der Theorie) Endstoff I vom Kp 71 bis 101 °C/0,13 mbar und $n_D^{25}$ = 1,5009.

Beispeil 2

a) 39,6 Teile Pyridin werden bei 10 °C unter Rühren in eine Mischung von 47,2 Teilen Chlorameisensäuremethylester und 78,8 Teile Isopropylsulfamidsäurechlorid in 330 Teilen Methylenchlorid innerhalb 15 Minuten eingegeben. Das Reaktionsgemisch wird 10 Minuten bei 22 °C nachgerührt. Nun wird das Reaktionsgemisch während 8 Minuten mit 350 Teilen Wasser bei 6 °C und einem pH von 1 gerührt. Dann wird aus dem gebildeten 2-phasigen Gemisch die organische Phase abgetrennt, filtriert und fraktioniert destilliert. Man erhält 72,1 Teile (67% der Theorie) N-Methoxycarbonyl-N-isopropylsulfamidsäurechlorid vom Kp 60 bis 64 °C/0,13 mbar und $n_D^{25}$ = 1,4540.

b) (Vergleich):

Die Umsetzung wird analog Beispiel 2a), aber ohne Wasserbehandlung analog Beispiel 1b) durchgeführt. Man erhält 14,5 Teile (13,5% der Theorie) sehr verunreinigten Endstoff I vom Kp 60 bis 80 °C/0,13 mbar und $n_D^{25}$ = 1,4912.

c) (Vergleich):

Die Umsetzung wird analog Beispiel 2a), aber ohne Wasserbehandlung durchgeführt. Das Reaktionsgemisch wird 10 Minuten bei Raumtemperatur nachgerührt. Nach Filtration, Nachwaschen des Filterrückstands mit Methylenchlorid und Filtrieren über Hyflo super cel-Filter wird der Rückstand im Vakuum eingeengt. Man erhält 16,1 Teile (15% der Theorie) sehr verunreinigten Endstoff I vom Kp 72 bis 83 °C/0,13 mbar und $n_D^{25}$ = 1,5022.

Beispiel 3

158,2 Teile Pyridin werden bei −10 °C in eine Lösung von 188,8 Teilen Chlorameisensäuremethylester in 1200 Teilen Methylenchlorid unter Rühren bei −10 °C eingegeben. Anschliessend werden bei gleicher Temperatur unter Rühren 259,2 Teile Methylsulfamidsäurechlorid zugegeben und das Gemisch eine halbe Stunde bei 0 °C und eine Stunde bei 25 °C gerührt. Nun wird das Reaktionsgemisch während 6 Minuten mit 600 Teilen Wasser bei 5 °C und einem pH von 1 gerührt. Dann wird aus dem gebildeten 2-phasigen Gemisch die organische Phase abgetrennt, filtriert und fraktioniert destilliert. Man erhält 341 Teile

(91% der Theorie) N-Methoxycarbonyl-N-methylsulfamidsäurechlorid mit Kp 49 bis 51 °C/0,13 mbar und $n_D^{25}$ = 1,4600.

Beispiele 4 bis 7 (Tabelle 1)

Analog Beispiel 3 werden die folgenden Verbindungen erhalten.

Beispiel 8

316,4 Teile Pyridin werden bei −10 °C unter Rühren in eine Lösung von 400 Teilen Phosgen in 2500 Teilen 1,2-Dichloräthan eingegeben. Bei gleicher Temperatur werden anschliessend 630,4 Teile Isopropylsulfamidsäurechlorid zugegeben und das Reaktionsgemisch noch eine Stunde bei 22 °C nachgerührt. Nun wird das Reaktionsgemisch während 8 Minuten mit 700 Teilen Wasser bei 6 °C und einem pH von 1 gerührt. Dann wird aus dem gebildeten 2phasigen Gemisch die organische Phase abgetrennt, filtriert und fraktioniert destilliert. Man erhält 763 Teile (87% der Theorie) N-Chlorcarbonyl-N-isopropylsulfamidsäurechlorid mit Kp 57 bis 62 °C/0,11 mbar und $n_D^{25}$ = 1,4749.

Beispiele 9 bis 12 (Tabelle 2)

Analog Beispiel 8 werden die folgenden Verbindungen erhalten:

Beispiel 13

197,8 Teile Pyridin werden unter Rühren bei −10 °C in eine Lösung von 196,3 Teilen Acetylchlorid in 1350 Teilen Methylenchlorid innerhalb 20 Minuten eingeführt. Anschliessend werden 359 Teile Äthylsulfamidsäurechlorid bei gleicher Temperatur zugegeben. Das Reaktionsgemisch wird eine halbe Stunde bei 0 °C und eine Stunde bei 22 °C nachgerührt. Nun wird das Reaktionsgemisch während 5 Minuten mit 800 Teilen Wasser bei 9 °C und einem pH von 1 gerührt. Dann wird aus dem gebildeten 2phasigen Gemisch die organische Phase abgetrennt, filtriert und fraktioniert destilliert. Man erhält 367 Teile (79% der Theorie) N-Acetyl-N-äthyl-sulfamidsäurechlorid mit Kp 34 bis 40 °C/0,11 mbar und $n_D^{25}$ = 1,4595.

Beispiele 14 bis 19 (Tabelle 3)

Auf gleiche Weise wie Beispiel 13 werden die folgenden Verbindungen erhalten:

Beispiel 20

79,1 Teile Pyridin werden unter Rühren bei 15 °C zu einer Lösung von 139,5 Teilen Oxalsäuredichlorid in 500 Teilen Toluol gegeben. Dann werden unter Eiskühlung 157,6 Teile Isopropylsulfamidsäurechlorid zugegeben und eine Stunde bei 22 °C nachgerührt. Nun wird das Reaktionsgemisch während 5 Minuten mit 200 Teilen Wasser bei 8 °C und einem pH von 1 gerührt. Dann wird aus dem gebildeten 2phasigen Gemisch die organische Phase abgetrennt, filtriert und fraktioniert destilliert. Man erhält 151 Teile (61% der Theorie) N-Chloroxalyl-N-isopropylsulfamidsäurechlorid mit Kp 70 bis 72 °C/2 mbar und $n_D^{25}$ = 1,4723.

Tabelle 1

| Beispiel | Teile | Ausgangsstoff III | Teile $RNHSO_2Cl$ | Ausgangsstoff II R | Teile Pyridin | Teile | Endstoff I | Kp (°C)/mbar | $n_D^{25}$ | Ausbeute (% der Theorie) |
|---|---|---|---|---|---|---|---|---|---|---|
| | | 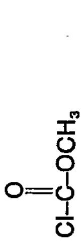 | | | | | 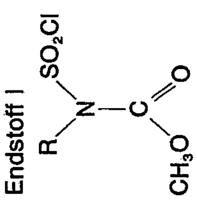 | | | |
| 4 | 330,4 | | 502,6 | $C_2H_5$ | 277,2 | 634 | | 50–60/0,1 | 1,4575 | 90 |
| 5 | 189,0 | | 315,2 | $nC_3H_7$ | 150,2 | 354 | | 120/0,13[a] | 1,4628 | 82 |
| 6 | 47,2 | | 89,0 | $Cl{-}CH_2{-}CH_2$ | 39,6 | 101,5 | | 84–88/0,11 | 1,4851 | 86 |
| 7 | 24,8 | | 50,5 | 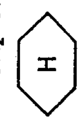 | 20,7 | 33,1 | | 91/0,12 | 1,4845 | 50 |

[a] Dünnschichtverdampfer

Tabelle 2

| Beispiel | Teile | Ausgangsstoff III | Teile RNHSO$_2$Cl | Ausgangsstoff II R | Teile | Base | Teile | Endstoff I | Kp (°C)/mbar | $n_D^{25}$ | Ausbeute (% der Theorie) |
|---|---|---|---|---|---|---|---|---|---|---|---|
| | | 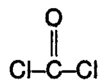 | | | | | | 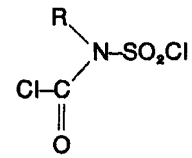 | | | |
| 9 | 328 | | 368 | $CH_3$ | 224,6 | Pyridin | 447,2 | | 49–51/0,11 | 1,4861 | 82 |
| 10 | 345 | | 430,7 | $C_2H_5$ | 237 | Pyridin | 525,4 | | 57/0,12 | 1,4798 | 85 |
| 11 | 205 | | 315,2 | $nC_3H_7$ | 186,2 | α-Picolin | 343,3 | | 67–72/0,12 | 1,4770 | 78 |
| 12 | 345 | | 514,8 | $nC_4H_9$ | 382 | H,H-Dimethyl-cyclohexylamin | 480 | | 70–76/0,12 | 1,4769 | 68,4 |

Tabelle 3

| Beispiel | Teile | Ausgangsstoff III | Teile | Ausgangsstoff II | Teile Pyridin | Teile | Endstoff I | Kp (°C)/mbar | $n_D^{25}$ | Ausbeute (% der Theorie) |
|---|---|---|---|---|---|---|---|---|---|---|
| 14 | 39,3 | 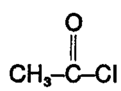 | 64,8 | $CH_3NHSO_2Cl$ | 39,6 | 72,2 | 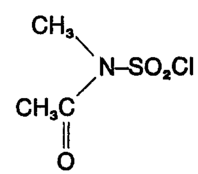 | 75/17 | 1,4668 | 84 |
| 15 | 39,3 | 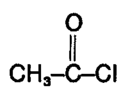 | 78,8 | $nC_3H_7NHSO_2Cl$ | 39,6 | 80,8 | 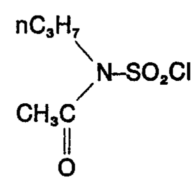 | 45/0,11 | 1,4610 | 81 |
| 16 | 39,3 | 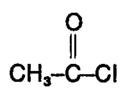 | 78,8 | $IC_3H_7NHSO_2Cl$ | 39,6 | 78,6 | 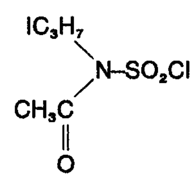 | 75–83/8,4 | 1,4608 | 79 |

| | | | | | | | | | | |
|---|---|---|---|---|---|---|---|---|---|---|
| 17 | 39,3 | 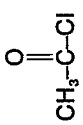 | 85,8 | $nC_4H_9NHSO_2Cl$ | 39,6 | 81,1 | 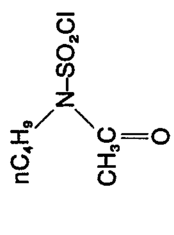 | 59–62/0,11 | 1,4616 | 76 |
| 18 | 39,3 | 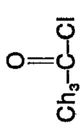 | 89,0 | $Cl–CH_2–CH_2NHSO_2Cl$ | 39,6 | 85,8 | 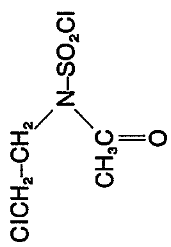 | 73–75/0,11 | 1,4922 | 78 |
| 19 | 98,4 | 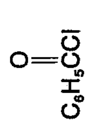 | 100 | $C_2H_5NHSO_2Cl$ | 55,4 | 114 | 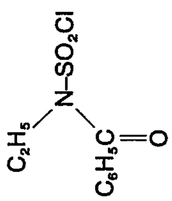 | 87–103/0,11 | 1,5368 | 69 |

Beispiele 21 bis 29 (Tabelle 4)

Analog Beispiel 20 werden die folgenden Verbindungen erhalten.

Beispiel 30

39,6 Teile Pyridin werden bei −10 °C unter Rühren zu einer Lösung von 62,3 Teilen Chlorameisensäurethioäthylester in 300 Teilen 1,2-Dichloräthan gegeben. Bei gleicher Temperatur werden dann 64,8 Teile Methylsulfamidsäurechlorid innerhalb 20 Minuten zugegeben und noch 45 Minuten bei 22 °C nachgerührt. Anschliessend wird das Reaktionsgemisch während 5 Minuten mit 150 Teilen Wasser bei 11 °C und einem pH von 1 gerührt. Dann wird aus dem gebildeten zweiphasigen Gemisch die organische Phase abgetrennt, filtriert und destilliert. Man erhält 97,4 Teile (89,5% der Theorie) N-Äthylmercaptocarbonyl-N-methylsulfamidsäurechlorid mit Kp 75 bis 78 °C/0,12mbar und $n_D^{25}$ = 1,5078.

Beispiel 31

Analog Beispiel 30 erhält man bei Einsatz von 39,6 Teilen Pyriden, 69,3 Teilen Chlorameisensäure-thio-n-propylester und 85,5 Teilen n-Butylsulfamidsäurechlorid nach der Destillation am Dünnschichtverdampfer (140 °C/0,12 mbar) 101 Teile (73,8% der Theorie) N-n-Propylmercaptocarbonyl-N-n-butylsulfamidsäurechlorid mit $n_D^{25}$ = 1,5000.

Beispiel 32 (Verwendung)

a) 26,3 Teile N-Methoxycarbonyl-N-methylsulfamidsäurechlorid aus Beispiel 3 und 18,2 Teile Triäthylamin werden gleichmässig über zwei Tropftrichter bei 22 °C unter Rühren zu einer Lösung von 41,5 Teilen 3,5-Dibrom-4-hydroxybenzonitril in 1000 Teilen Acetonitril gegeben. Die Reaktionsmischung wird eine Stunde bei 60 °C nachgerührt, im Vakuum eingeengt und dann in 400 Teilen Methylenchlorid aufgenommen. Nach der Extraktion mit 200 Volumenteilen 2-n-Natronlauge, Trocknen der organischen Phase über Magnesiumsulfat, Chromatographie über neutralem Aluminiumoxid und Einengen im Vakuum erhält man das 2,6-Dibrom-4-cyanophenyl-1-N-methoxycarbonyl-N-methylaminosulfonat mit Fp 93 bis 96 °C

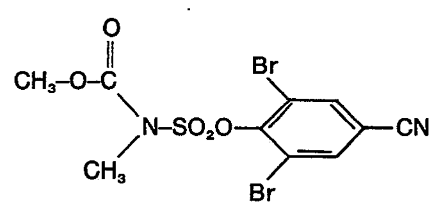

b) Die herbizide Wirksamkeit des Endstoffs von Beispiel 32a) wird anhand von Gewächshausversuchen festgestellt.

Als Kulturgefässe dienten Plastikblumentöpfe mit 300 cm³ Inhalt, die mit lehmigem Sand mit etwa 1,5% Humus gefüllt wurden. Die Samen der Testpflanzen werden nach Arten getrennt flach eingesät. Unmittelbar danach erfolgt bei Vorauflaufbehandlung das Aufbringen der Wirkstoffe auf

Tabelle 4

| Beispiel | Teile | Ausgangsstoff III | Teile | Ausgangsstoff II | Teile Pyridin | Teile | Endstoff I | Kp (°C)/mbar | $n_D^{25}$ | Ausbeute (% der Theorie) |
|---|---|---|---|---|---|---|---|---|---|---|
| 21 | 27,9 | 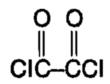 | 25,9 | $CH_3NHSO_2Cl$ | 15,8 | 26 | 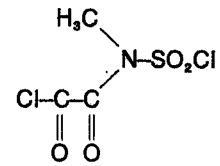 | 60–65/2 | 1,4834 | 58 |
| 22 | 27,9 | 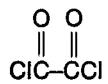 | 28,7 | $C_2H_5NHSO_2Cl$ | 15,8 | 20 | 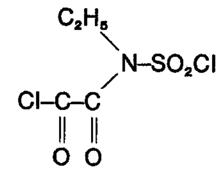 | 70–72/0,4 | 1,4790 | 42 |
| 23 | 139,5 | 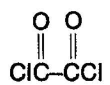 | 157,6 | $(n)C_3H_7NHSO_2Cl$ | 79,1 | 107 | 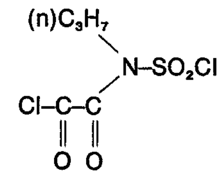 | 75–84/0,66 | 1,4745 | 43 |
| 24 | 139,5 | 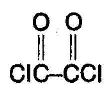 | 171,6 | $(n)C_4H_9NHSO_2Cl$ | 79,1 | 142 | 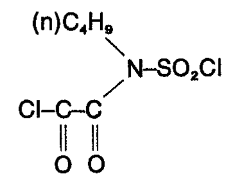 | 87–94/0,66 | 1,4720 | 54 |
| 25 | 30 | 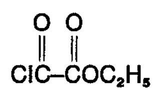 | 25,9 | $CH_3NHSO_2Cl$ | 15,8 | 26 | 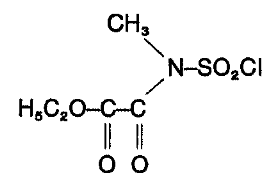 | 80–82/0,66 | 1,4587 | 61 |
| 26 | 30 | 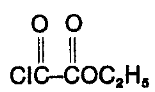 | 28,7 | $H_5C_2NHSO_2Cl$ | 15,8 | 34 | 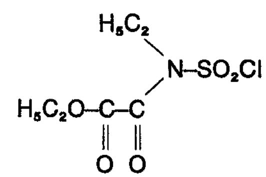 | 75–83/0,52 | 1,4579 | 70 |

| | | | | | | | | | | |
|---|---|---|---|---|---|---|---|---|---|---|
| 27 | 30 | 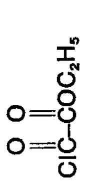 | 31,5 | (n)$C_3H_7NHSO_2Cl$ | 15,8 | 30,8 | 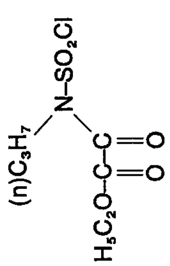 | 82–85/0,52 | 1,4573 | 60 |
| 28 | 150,1 | 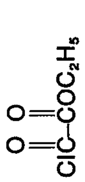 | 157,6 | (I)$C_3H_7NHSO_2Cl$ | 79,1 | 193 | 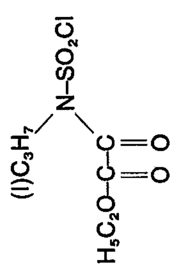 | 78–82/0,52 | 1,4539 | 75 |
| 29 | 30 | 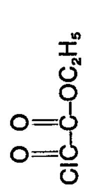 | 34,3 | (n)$C_4H_9NHSO_2Cl$ | 15,8 | 36 | 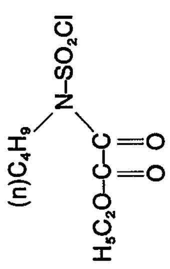 | 100–110/0,66 | 1,4588 | 70 |

die Erdoberfläche. Sie werden hierbei in Wasser als Verteilungsmittel suspendiert oder emulgiert und mittels fein verteilender Düsen auf die Erde gespritzt. Die Aufwandmengen betragen jeweils 3,0 kg/ha Aktivsubstanz. Nach dem Aufbringen der Mittel werden die Töpfe leicht beregnet, um Keimung und Wachstum der Pflanzen anzuregen und gleichzeitig die chemischen Mittel zu aktivieren. Danach deckt man die Gefässe mit durchsichtigen Plastikhauben ab bis die Pflanzen angewachsen sind. Diese Abdeckung bewirkt ein gleichmässiges Keimen der Testpflanzen.

Zum Zwecke der Nachauflaufbehandlung zieht man die Pflanzen je nach Wuchsform in den Versuchsgefässen erst bis zu einer Höhe von 3 bis 10 cm an und behandelt sie dann. Auch hier beträgt die Dosis 3 kg/ha Aktivsubstanz. Eine Abdeckung unterbleibt. Die Versuche erfolgen im Gewächshaus, wobei für wärmeliebende Arten wärmere Bereiche des Gewächshauses (25 bis 40 °C) und für solche gemässigter Klimate 15 bis 30 °C verwendet werden. Die Versuchsperiode erstreckte sich über 4 bis 6 Wochen.

Während dieser Zeit werden die Pflanzen gepflegt und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Sprossteile.

Bei den als Indikatorpflanzen eingesetzten Arten Sinapis alba, zeigt sich eine totale Wirkung bei Vorauflaufanwendung. Ipomoea spp. und Centaurea cyanus werden bei Nachauflaufanwendung abgetötet. Die Kulturpflanzen Hafer (Avena sativa) und Deutsches Weidelgras (Lolium multiflorum) bleiben bei beiden Behandlungsarten schadfrei. Die Ergebnisse zeigen im Vergleich zu bekannten, für diesen Verwendungszweck bisher benutzten Stoffen eine deutlich bessere Wirkung bei der Bekämpfung von unerwünschten breitblättrigen Pflanzen in Kulturen aus der Familie der Gramineen.

Beispiel 33

55,4 Teile Pyridin werden bei −5 °C unter Rühren in eine Mischung von 112,4 Teilen Chlorcarbonylisocyaniddichlorid in 700 Teilen 1,2-Dichloräthan eingegeben. Dann werden bei 0 °C innerhalb 15 Minuten 110,3 Teile n-Propylsulfamidsäurechlorid zugegeben. Nach 1 ½ Stunden Rühren bei 22 °C wird das Reaktionsgemisch abgesaugt und das Filtrat während 5 Minuten mit 300 Teilen Wasser bei 5 °C und einem pH von 1 gerührt. Dann wird aus dem gebildeten 2phasigen Gemisch die organische Phase abgetrennt, filtriert und fraktioniert destilliert. Man erhält 124,7 Teile (63% der Theorie) N-Propyl-N-carbonylisocyaniddichlorido-sulfamidsäurechlorid vom Kp 95 bis 98 °C/0,13 mbar und $n_D^{25}$ = 1,4972.

Beispiel 34 bis 37 (Tabelle 6)

Analog Beispiel 33 werden die folgenden Verbindungen erhalten.

Tabelle 6

| Beispiel | Teile | Ausgangsstoff III | Teile RNHSO$_2$Cl | Ausgangsstoff II R | Teile Pyridin | Teile | Endstoff I | Kp (°C)/mbar) | $n_D^{25}$ | Ausbeute (% der Theorie) |
|---|---|---|---|---|---|---|---|---|---|---|
| | | 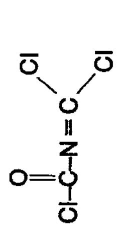 | | | | | 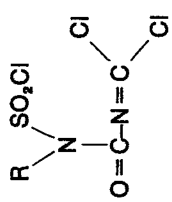 | | | |
| 34 | 32,2 | | 25,8 | $CH_3$ | 15,8 | 36,1 | | 75–82/0,02 | 1,5038 | 71,2 |
| 35 | 32,2 | | 28,7 | $C_2H_5$ | 15,8 | 37,5 | | 68–75/0,02 | 1,4968 | 70,1 |
| 36 | 32,2 | | 31,5 | $C_3H_7$(i) | 15,8 | 39,1 | | 66/0,02 | 1,4981 | 69,4 |
| 37 | 32,2 | | 34,3 | $C_4H_9$(n) | 15,8 | 34,2 | | 88–95/0,02 | 1,4929 | 57,8 |

Beispiel 38 (Verwendung)

a) Zu 19,9 Teilen N-Carbonylisocyanato-N-methyl-sulfamidsäurechlorid aus Beispiel 2 in 150 Teilen Äther werden bei −50 °C bis −55 °C 6 Teile Isopropylamin in 50 Teilen Äther portionsweise zugefügt. Man kühlt das gesamte Reaktionsgemisch auf Raumtemperatur, saugt den gebildeten Niederschlag ab, behandelt ihn mit Eiswasser und saugt ab. Man erhält 14 Teile (54% der Theorie) N-Isopropyl-N'-(N''-methyl-N''-chlorsulfonyl)-carbamidoharnstoff mit Fp 100 bis 105 °C.

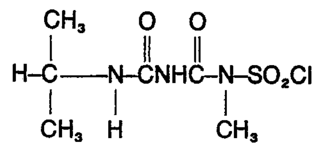

b) Die herbizide Wirksamkeit des Endstoffs von Beispiel 38a) wird anhand von Gewächshausversuchen festgestellt.

Als Kulturgefässe dienten Plastikblumentöpfe mit 300 cm³ Inhalt, die mit lehmigem Sand mit etwa 1,5% Humus gefüllt wurden. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierbei in Paraffinöl als Verteilungsmittel suspendiert oder emulgiert und mittels fein verteilender Düsen auf die Erde gespritzt. Die Aufwandmengen betragen jeweils 3,0 kg/ha Aktivsubstanz. Nach dem Aufbringen der Mittel werden die Töpfe leicht beregnet, um Keimung und Wachstum der Pflanzen anzuregen und gleichzeitig die chemischen Mittel zu aktivieren. Danach deckt man die Gefässe mit durchsichtigen Plastikhauben ab bis die Pflanzen angewachsen sind. Diese Abdeckung bewirkt ein gleichmässiges Keimen der Testpflanzen.

Zum Zwecke der Nachauflaufbehandlung zieht man die Pflanzen je nach Wuchsform in den Versuchsgefässen erst bis zu einer Höhe von 3 bis 10 cm an und behandelt sie dann. Auch hier beträgt die Dosis 3 kg/ha Aktivsubstanz. Eine Abdeckung unterbleibt. Die Versuche erfolgen im Gewächshaus, wobei für wärmeliebende Arten wärmere Bereiche des Gewächshauses (25 bis 40 °C) und für solche gemässigter Klimate 15 bis 30 °C verwendet werden. Die Versuchsperiode erstreckte sich über 3 bis 6 Wochen.

Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Sprossteile.

Die Prüfung ergibt, dass der Endstoff I aus Beispiel 38a) bei Vorauflaufanwendung eine herbizide Wirkung an Hafer (Avena sativa) als ein Vertreter für grasartige Pflanzen zeigt. Bei Nachauflaufanwendung werden einjähriges Weidelgras (Lolium multiflorum) und Prunkwindearten (Ipomoea spp.) – letztere als Vertreter breitblättriger unerwünschter Pflanzen – stark geschädigt.

## Patentansprüche

1. Verfahren zur Herstellung von Sulfamidsäurehalogeniden der Formel

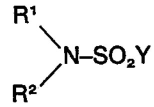

I,

in der $R^1$ einen aliphatischen oder cycloaliphatischen Rest bedeutet, $R^2$ den Rest

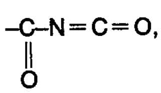, 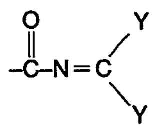, 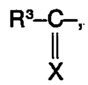, 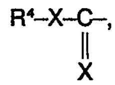, 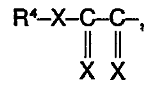, 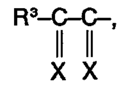, 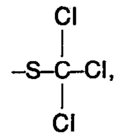, 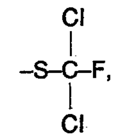,

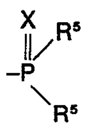 $-CH_2XR^6$, 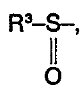, $R^3-S-S-$ oder 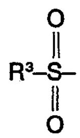

bezeichnet, $R^3$ und $R^4$ jeweils für einen aliphatischen, cycloaliphatischen, araliphatischen, aromatischen oder heterocyclischen Rest stehen, $R^3$ auch ein Halogenatom bezeichnen kann, die einzelnen Reste $R^5$ gleich oder verschieden sein können und jeweils ein Halogenatom, den Rest $-X-R^6$ oder $-R^6$ bedeuten, $R^6$ für einen aliphatischen Rest steht, die einzelnen Reste X gleich oder verschieden sein können und jeweils ein Sauerstoffatom oder ein Schwefelatom bedeuten, die einzelnen Reste Y gleich oder verschieden sein können und jeweils für ein Halogenatom stehen, durch Umsetzung von Sulfamidsäurehalogeniden und Halogenverbindungen in Gegenwart von Basen und Lösungsmitteln, dadurch gekennzeichnet, dass man monosubstituierte Sulfamidsäurehalogenide der Formel

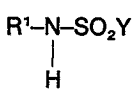

II,

in der $R^1$ und Y die vorgenannten Bedeutungen besitzen, mit Halobenverbindungen der Formel

$$R^2-Y \quad III,$$

in der $R^2$ und Y die vorgenannten Bedeutungen besitzen, in Gegenwart einer basischen Verbindung in einer Menge von 1 bis 1,5 Äquivalenten je Mol Ausgangsstoff II und von inerten, organischen Lösungsmitteln umsetzt und anschliessend das so gebildete Reaktionsgemisch mit Wasser bei einem pH von höchstens 7 behandelt.

2. Sulfonamidsäurehalogenide der Formel

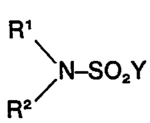

I,

in der $R^1$ einen Alkylrest oder einen durch mehrere Fluor- und/oder Chloratome, oder durch ein Fluoratom oder Chloratom in β-, γ- und/oder δ-Stellung zum Stickstoffatom substituierten Alkylrest mit jeweils 1 bis 8 Kohlenstoffatomen oder einen Cycloalkylrest mit 4 bis 8 Kohlenstoffatomen bedeutet, $R^2$ den Rest

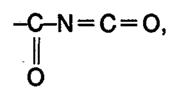, 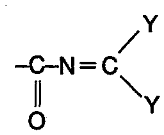, 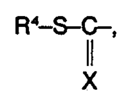,

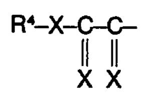 oder 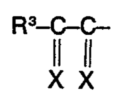

bezeichnet, darüber hinaus, wenn Y Chlor bedeutet, $R^2$ auch für 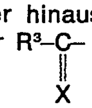 stehen kann, $R^3$ und $R^4$ jeweils für einen unsubstituierten oder einen durch ein oder zwei Fluor- oder Chloratome substituierten Alkylrest mit jeweils 1 bis 8 Kohlenstoffatomen, oder einen gegebenenfalls durch ein oder 2 Halogenatome, Nitro- und/oder Alkoxygruppen mit 1 bis 3 Kohlenstoffatomen substituierten Arylrest mit 6 bis 12 Kohlenstoffatomen stehen, $R^3$ auch, wenn Y Chlor und $R^1$ Äthyl, n-Propyl, n-Butyl, sek.-Butyl bedeuten, ein Chloratom bezeichnen kann, die einzelnen Reste X gleich oder verschieden sein können und jeweils ein Sauerstoffatom oder ein Schwefelatom bedeuten, die einzelnen Reste Y gleich oder verschieden sein können und jeweils für ein Fluor- oder Chloratom stehen.

## Claims

1. A process for the preparation of a sulfamic acid halide of the formula

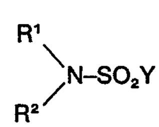

I,

where $R^1$ is an aliphatic or cycloaliphatic radical, $R^2$ is

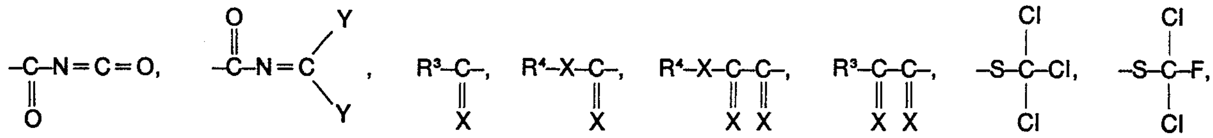

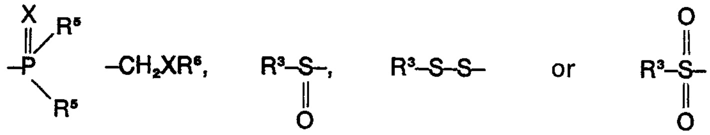

or

$R^3$ and $R^4$ are each an aliphatic, cycloaliphatic, araliphatic, aromatic or heterocyclic radical, $R^3$ may also be halogen, the individual radicals $R^5$ may be identical or different and each is halogen, $-X-R^6$ or $-R^6$, $R^6$ is an aliphatic radical, the individual radicals X may be identical or different and each is oxygen or sulfur, and the individual radicals Y may be identical or different and each is halogen, by reacting a sulfamic acid halide and a halogen compound in the presence of a base and a solvent, characterized in that a monosubstituted sulfamic acid halide of the formula

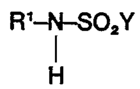 II,

where $R^1$ and Y have the above meanings, is reacted with a halogen compound of the formula

$$R^2-Y \quad III$$

where $R^2$ and Y have the above meanings, in the presence of from to 1.5 equivalents of a basic compound per mole of starting material II, and of an inert, organic solvent, and the resulting reaction mixture is subsequently treated with water at a pH of at most 7.

2. A sulfamic acid halide of the formula

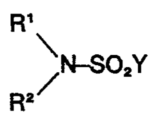 I,

where $R^1$ is alkyl of 1 to 8 carbon atoms optionally substituted by several fluorine and/or chlorine atoms or by one fluorine or chlorine atom in the β-, γ- and/or δ-position to the nitrogen, or is cycloalkyl of 5 to 8 carbon atoms, $R^2$ is

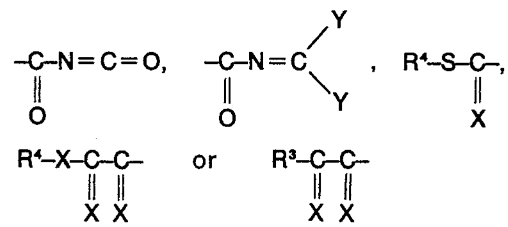

or

or, if Y is chlorine, $R^2$ may also denote 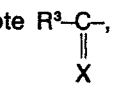

$R^3$ and $R^4$ are each alkyl of 1 to 8 carbon atoms optionally substituted by one or two fluorine or chlorine atoms, or aryl of 6 to 12 carbon atoms optionally substituted by one or two halogen atoms, nitro groups and/or alkoxy groups of 1 to 3 carbon atoms, or, if Y is chlorine and $R^1$ is ethyl, n-propyl, n-butyl or sec-butyl, $R^3$ may also denote chlorine, the individual radicals X may be identical or different and each is oxygen or sulfur, and the individual radicals Y may be identical or different and each is fluorine or chlorine.

**Revendications**

1. Procédé pour la préparation d'halogénures d'acides sulfamiques de formule

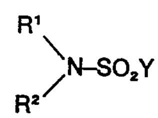 I,

dans laquelle $R^1$ représente un radical aliphatique ou cycloaliphatique, $R^2$ le radical

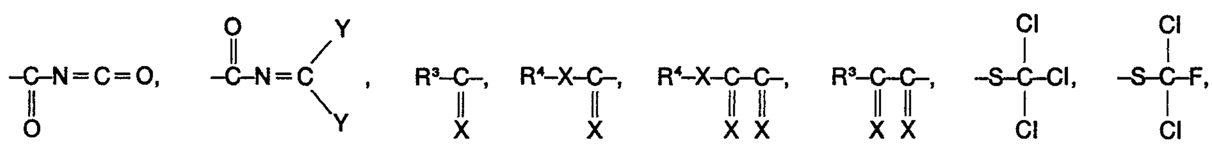

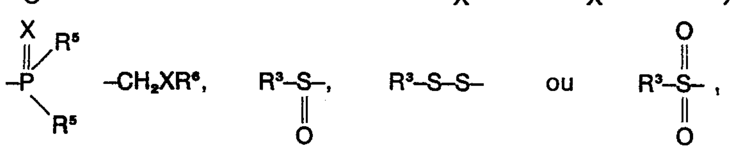

ou

, $R^3$ et $R^4$

étant mis chacun pour un radical aliphatique, cycloaliphatique, araliphatique, aromatique ou hétérocyclique, $R^3$ pouvant également désigner un atome d'halogène, les différents radicaux $R^5$ pouvant être semblables ou différents et représentant chacun un atome d'halogène, le radical $-X-R^6$ ou $-R^6$, $R^6$ étant mis pour un radical aliphatique, les différents radicaux X pouvant être semblables ou différents et représentant chacun un atome d'oxygène ou un atome de soufre, les différents radicaux Y pouvant être semblables ou différents et étant mis chacun pour un atome d'halogène, par réaction d'halogénures d'acides sulfamiques et de composés halogénés en

présence de bases et de solvants, caractérisé en ce qu'on réagir des halogénures d'acides sulfamiques monosubstitués de formule

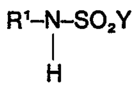 II,

dans laquelle $R^1$ et Y ont les significations données ci-dessus, avec des composés halogénés de formule

$R^2$–Y (III)

dans laquelle $R^2$ et Y ont les significations données ci-dessus, en présence d'un composé basique, dans une proportion de 1 à 1,5 équivalents par mol de substance de départ II, et de solvants organiques inertes, puis on traite le mélange réactionnel ainsi formé par l'eau à un pH de 7 au maximum.

2. Halogénures d'acides sulfamiques de formule

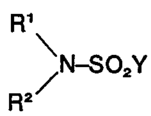 I,

dans laquelle $R^1$ représente un radical alcoyle à 1–8 atomes de carbone, non substitué ou substitué par plusieurs atomes de fluor et/ou de chlore ou par un atome de fluor ou un atome de chlore en position β, γ et/ou δ par rapport à l'atome d'azote, ou un radical cycloalcoyle à 4–8 atomes de carbone, $R^2$ représente le radical

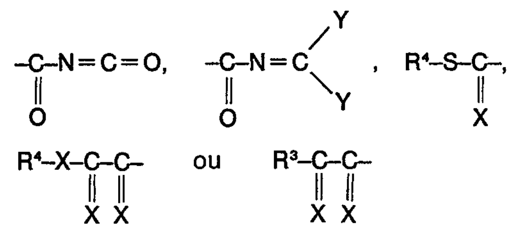

et en outre, lorsque Y représente un chlore, $R^2$ peut être également mis pour 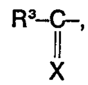, $R^3$ et $R^4$ étant mis chacun pour un radical alcoyle à 1–8 atomes de carbone, non substitué ou substitué par un ou deux atomes de fluor ou de chlore, ou pour un radical aryle à 6–12 atomes de carbone, éventuellement substitué par un ou deux atomes d'halogènes, des groupes nitro et/ou alcoxy à 1–3 atomes de carbone, $R^3$ pouvant également représenter un atome de chlore lorsque Y est un chlore et $R^1$ und éthyle, un n-propyle, un n-butyle ou un butyle secondaire, les différents radicaux X pouvant être semblables ou différents et représentant chacun un atome d'oxygène ou un atome de soufre, les différents radicaux Y pouvant être semblables ou différents et étant mis chacun pour un atome de fluor ou de chlore.